# EUROPEAN PATENT APPLICATION

(11) **EP 4 632 050 A1**
(43) Date of publication of application: **15.10.2025**
(21) Application number: 24741562.3
(22) Date of filing: 11.01.2024
(51) Int. Cl.: C10M 105/42, C09K 5/04, C10M 105/38, C10N 30/00, C10N 30/08, C10N 40/30

(54) **COMPLEX ESTER, REFRIGERATOR OIL, AND WORKING FLUID COMPOSITION**

(30) Priority: 13.01.2023 JP 2023003776
(71) Applicant: ENEOS Corporation, Chiyoda-ku Tokyo 100-8162 (JP)
(72) Inventor: KAWAGUCHI, Masaki, Tokyo 100-8162 (JP); OKAZAKI, Motoya, Tokyo 100-8162 (JP); MIZUTANI, Yuya, Tokyo 100-8162 (JP); OGATA, Hidetoshi, Tokyo 100-8162 (JP); SEKI, Yuma, Tokyo 100-8162 (JP)
(74) Representative: Grünecker Patent- und Rechtsanwälte PartG mbB
(86) International application number: PCT/JP2024/000416
(87) International publication number: WO 2024/150790

(57) **Abstract**

A complex ester of a divalent carboxylic acid, a divalent alcohol, and at least one selected from a monohydric alcohol and a monocarboxylic acid, wherein the divalent alcohol consists of a branched divalent alcohol having no quaternary carbon.

## Description

### Technical Field

The present invention relates to a complex ester, a refrigerating machine oil, and a working fluid composition.

### Background Art

Refrigerating machines such as refrigerators, car air conditioners, room air conditioners, and vending machines include compressors for circulating a refrigerant in a refrigeration cycle. The compressor is filled with a refrigerating machine oil for lubricating sliding members. Refrigerating machine oil generally comprises a base oil and additives selected according to the desired properties.

As a base oil, for example, esters such as polyol esters and complex esters may be used. Patent Literature 1, for instance, discloses a complex ester with excellent lubricity and heat resistance, obtained from (A) neopentyl glycol, (B) a straight-chain divalent alcohol having 2 to 6 carbon atoms and hydroxyl groups at both terminal carbons, (C) a straight-chain divalent carboxylic acid having 4 to 10 carbon atoms and carboxyl groups at both terminal carbons, and (D) a monohydric alcohol having 6 to 12 carbon atoms.

### Citation List

### Patent Literature

[Patent Literature 1] Japanese Patent No. 6614510

### Summary of Invention

### Technical Problem

Under conditions where the refrigerant and the refrigerating machine oil separate from each other, such as at startup of the compressor, an oil-poor layer having a significantly low-viscosity in the oil reservoir at the bottom of the compressor may be supplied to each sliding portion. Therefore, if it is possible to use a base oil with as high a density as possible (for example, a base oil having a density equal to or higher than that of the refrigerant), it could potentially improve lubrication at compressor startup. Further, it is required that a base oil used for refrigerating machine oil exhibit good low-temperature fluidity and be less prone to precipitation at low temperatures (that is, have excellent low-temperature stability). However, conventional complex esters are not always sufficient in meeting such requirements, and there is still room for improvement.

Accordingly, one aspect of the present invention is to provide a novel complex ester having a higher density and being excellent in low-temperature stability.

### Solution to Problem

The inventors have found that by using, as the divalent alcohol in a complex ester, only a branched divalent alcohol having no quaternary carbon, it becomes easier to achieve both high density and low-temperature stability compared to a conventional complex ester using neopentyl glycol or a straight-chain divalent alcohol.

The present invention includes the following aspects.
[1] A complex ester of a divalent carboxylic acid, a divalent alcohol, and at least one selected from a monohydric alcohol and a monocarboxylic acid, wherein the divalent alcohol consists of a branched divalent alcohol having no quaternary carbon.
[2] A refrigerating machine oil comprising a complex ester of a divalent carboxylic acid, a divalent alcohol, and at least one selected from a monohydric alcohol and a monocarboxylic acid,
   wherein the divalent alcohol consists only of a branched divalent alcohol having no quaternary carbon.
[3] The refrigerating machine oil according to [2], further comprising a polyol ester of a polyhydric alcohol and a fatty acid.
[4] A working fluid composition comprising:
   the refrigerating machine oil according to [2] or [3], and
   a refrigerant.
[5] The working fluid composition according to [4], wherein the refrigerant comprises difluoromethane.

### Advantageous Effects of Invention

According to one aspect of the present invention, it is possible to provide a novel complex ester having a higher density and being excellent in low-temperature stability.

### Description of Embodiments

One embodiment of the present invention is a complex ester of a divalent carboxylic acid, a divalent alcohol, and at least one selected from a monohydric alcohol and a monocarboxylic acid.

The divalent carboxylic acid may be, for example, a divalent carboxylic acid having 6 to 12 carbon atoms. Examples of the divalent carboxylic acid include adipic acid, pimelic acid, suberic acid, azelaic acid, sebacic acid, phthalic acid, and trimellitic acid. The divalent carboxylic acid is preferably at least one selected from adipic acid and sebacic acid, and more preferably adipic acid.

The divalent alcohol consists of a branched divalent alcohol having no quaternary carbon. In other words, the divalent alcohol comprises no divalent alcohol having a quaternary carbon, and comprises no straight-chain divalent alcohol. The divalent alcohol may be a divalent aliphatic alcohol. The carbon number of the divalent alcohol may be, for example, 3 or more, and may be 8 or less, 7 or less, or 6 or less.

A branched divalent alcohol having no quaternary carbon may be, for example, a divalent alcohol represented by the following formula (1): 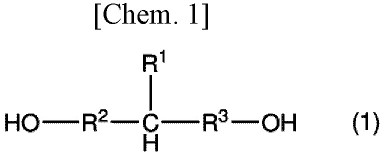 wherein R¹ represents a straight-chain alkyl group, and R² and R³ each independently represent a single bond or a straight-chain alkylene group.

The number of carbon atoms in the straight-chain alkyl group represented by R¹ may be 1 or more, and may be 3 or less, 2 or less, or 1. The number of carbon atoms in the straight-chain alkylene groups represented by R² and R³ may be 1 or more, and may be 3 or less, or 2 or less.

Examples of the branched divalent alcohol having no quaternary carbon include branched divalent alcohols having an alkyl group of 1 to 3 carbon atoms, such as 1,2-propanediol (1,2-propylene glycol), 2-methyl-1,3-propanediol, 2-methyl-1,4-butanediol, 3-methyl-1,5-pentanediol, 1,2-butanediol, 1,3-butanediol, 1,2-pentanediol, 1,3-pentanediol, and 1,4-pentanediol.

The monohydric alcohol may be a monohydric aliphatic alcohol. The number of carbon atoms in the monohydric alcohol may be 4 or more, 6 or more, or 8 or more, and may be 18 or less, 14 or less, or 10 or less. Examples of the monohydric alcohol include butanol, pentanol, hexanol, heptanol, octanol, nonanol, decanol, dodecanol, and oleyl alcohol. These monohydric alcohols may be linear or branched. From the viewpoint of further improving low-temperature properties of the complex ester, the monohydric alcohol is preferably a monohydric alcohol having 4 to 8 carbon atoms, and more preferably is selected from linear butanol, hexanol, and branched octanol, and further more preferably from 1-butanol and 2-ethylhexanol.

The number of carbon atoms in the monocarboxylic acid may be 2 or more, 5 or more, or 8 or more, and may be 12 or less, 11 or less, or 10 or less. Examples of the monocarboxylic acid include ethanoic acid, propanoic acid, butanoic acid, pentanoic acid, hexanoic acid, heptanoic acid, octanoic acid, nonanoic acid, decanoic acid, and dodecanoic acid. These monocarboxylic acid may be linear or branched. From the viewpoint of further improving low-temperature properties of the complex ester, the monocarboxylic acid is preferably selected from branched octanoic acid and branched nonanoic acid, and more preferably from 2-ethylhexanoic acid and 3,5,5-trimethylhexanoic acid.

When the "at least one selected from a monohydric alcohol and a monocarboxylic acid" is a monohydric alcohol, for example, the complex ester can be obtained by synthesizing an ester intermediate in which part of the carboxyl groups of the divalent carboxylic acid remain unesterified by adjusting the molar ratio of the divalent carboxylic acid to the divalent alcohol, and by esterifying the remaining carboxyl groups with the monohydric alcohol. The resulting complex ester may be, for instance, a complex ester represented by the following formula (2): 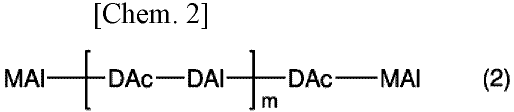 wherein DAc represents a residue obtained by removing the -OH groups from the two -COOH groups of the divalent carboxylic acid; DAl represents the residue obtained by removing the H atoms from the two - OH groups of the divalent alcohol; MAl represents the residue obtained by removing the H atom from the one -OH group of the monohydric alcohol; and m is an integer of 1 or more. The integer m may be, for example, 1 to 10, or 1 to 5. Adjacent MAl and DAc are bonded to each other by an ester bond, and adjacent DAc and DAl are bonded to each other by an ester bond.

The complex ester may comprise, as its main component (for example, in an amount of 50% by mass or more), the complex ester represented by formula (2), and may further comprise unavoidable byproducts. The complex ester preferably comprises, as its main component, the complex ester in which m is 1 to 10, and more preferably comprises, as its main component, the complex ester in which m is 1 to 5.

When the "at least one selected from a monohydric alcohol and a monocarboxylic acid" is a monocarboxylic acid, for example, the complex ester can be obtained by synthesizing an ester intermediate in which part of the hydroxyl groups of the divalent alcohol remain unesterified by adjusting the molar ratio of the divalent carboxylic acid to the divalent alcohol, and by esterifying the remaining hydroxyl groups with the monocarboxylic acid. This complex ester may be, for instance, a complex ester represented by the following formula (3): 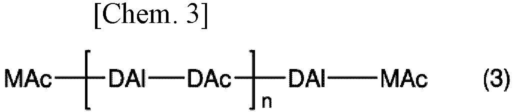 wherein DAc represents the residue obtained by removing the -OH groups from the two -COOH groups of the divalent carboxylic acid; DAl represents the residue obtained by removing the H atoms from the two - OH groups of the divalent alcohol; MAc represents the residue obtained by removing the OH group from the one -COOH group of the monocarboxylic acid; and n is an integer of 1 or more. The integer n may be, for example, 1 to 10, or 1 to 5. Adjacent MAc and DAl are bonded to each other by an ester bond, and adjacent DAl and DAc are bonded to each other by an ester bond.

The complex ester may comprise, as its main component (for example, in an amount of 50% by mass or more), the complex ester represented by formula (3), and may further comprise unavoidable byproducts. The complex ester preferably contains, as its main component, the complex ester in which n is 1 to 10, and more preferably comprises, as its main component, the complex ester in which n is 1 to 5.

From the viewpoint of obtaining more excellent stability, it is preferred that the "at least one selected from a monohydric alcohol and a monocarboxylic acid" be a monohydric alcohol; in other words, it is preferred that the complex ester be a complex ester of a divalent carboxylic acid, a divalent alcohol, and a monohydric alcohol.

In the above complex ester, by using only a branched divalent alcohol having no quaternary carbon as the divalent alcohol, it becomes easier to achieve both high density and low-temperature stability, compared to a case where the divalent alcohol is neopentyl glycol or a straight-chain divalent alcohol.

The density of the complex ester at 15°C is preferably 1.02 g/cm³ or more, 1.025 g/cm³ or more, 1.03 g/cm³ or more, 1.035 g/cm³ or more, or 1.04 g/cm³ or more. The density of the complex ester may also be 1.1 g/cm³ or less, 1.09 g/cm³ or less, or 1.08 g/cm³ or less.

Generally, as the kinematic viscosity increases, the density tends to increase. In one embodiment, for a complex ester having a kinematic viscosity at 40°C of less than 100 mm²/s, the density at 15°C may be 1.01 g/cm³ or more, preferably 1.02 g/cm³ or more; for a complex ester having a kinematic viscosity at 40°C of from 100 mm²/s to 180 mm²/s, the density at 15°C may be 1.02 g/cm³ or more, preferably 1.03 g/cm³ or more; and for a complex ester having a kinematic viscosity at 40°C of 180 mm²/s or more, or from 180 mm²/s to 500 mm²/s, the density at 15°C may be 1.03 g/cm³ or more, preferably 1.04 g/cm³ or more. The density mentioned herein is the density at 15°C measured by the "vibration method" described in JIS K2249-1:2011.

An example of the technical significance of higher density in the complex ester, more specifically, in the case of using the above complex ester together with R32 which is a refrigerant, is described below.

Generally, as the temperature rises, the density of a liquid tends to decrease. Meanwhile, the liquid density of the refrigerant used with the refrigerating machine oil has a much larger temperature dependence than the density of the refrigerating machine oil. Typically, at low temperatures, the refrigerant has a higher density than the refrigerating machine oil, but its density drops sharply as temperature rises, and in a certain temperature range it becomes less dense than the refrigerating machine oil. For example, in the case of R134a, the liquid density at 15°C is 1.24 g/cm³, and even at a high temperature, such as 70°C, it is not generally expected that the density of the refrigerant and the refrigerating machine oil would invert. In contrast, in the case of R32, the liquid density at 15°C is 1.00 g/cm³, and it can happen that, in the temperature range of 30°C or higher, the density of the refrigerant becomes lower than that of the refrigerating machine oil. In the combination of refrigerant and refrigeration oil where the temperature range in which the density of the refrigeration oil is higher than that of the refrigerant extends to the lower temperature side, when separated into two layers, the temperature range in which the oil-rich layer having a higher density is below the refrigerant-rich layer having a lower density will extend further to the lower temperature side. A compressor in a refrigerating machine has an oil reservoir at the bottom of the compressor, from which the refrigerating machine oil is drawn through an oil tube and supplied to sliding parts within the compressor to lubricate those parts. Under steady-state operation of a refrigerant compressor using R32, for example, a temperature range of 60°C or higher is often one in which the refrigerating machine oil is denser than the R32. As a result, an oil-rich layer exists in the oil reservoir, ensuring the formation of an oil film with optimal viscosity, thereby guaranteeing lubrication.

On the other hand, in daily use, when compressor operation is stopped and the system is left to stand at around ambient temperature of, for example, 30°C or lower, and then (for example, the next day) the compressor is restarted, if the refrigerant and the refrigerating machine oil have separated into two layers before startup, the oil reservoir may contain a refrigerant-rich layer having a high density. In this state, if the compressor is started, the refrigerant-rich layer having a low oil concentration is supplied to the sliding parts within the compressor, making it impossible to maintain an oil film with an appropriate viscosity and potentially causing wear or seizing of the sliding parts.

Therefore, when using a refrigerating machine oil that separates into two layers with the refrigerant at an ambient temperature range of 30°C or lower, it is preferable to reduce the above risk. As the aforementioned complex ester can have a density (at 15°C) higher than that of R32 in an ambient temperature region of 30°C or lower (e.g., 5°C to 25°C), if a two layer separation occurs, the bottom layer in the oil reservoir can be an oil-rich layer, thereby reducing the risk of troubles when the compressor is started.

The number average molecular weight (Mn) of the complex ester may be 300 or more, 500 or more, or 600 or more, and may be 3000 or less, 2000 or less, or 1500 or less. The weight average molecular weight (Mw) of the complex ester may be 400 or more, 600 or more, or 700 or more, and may be 5000 or less, 3000 or less, or 2000 or less. The ratio Mw/Mn (i.e., dispersity) of the weight average molecular weight to the number average molecular weight of the complex ester may be 1.3 or more, 1.5 or more, or 1.6 or more, and may be 3 or less, 2.5 or less, or 2 or less. The number average molecular weight Mn and weight average molecular weight Mw used herein refer to polystyrene-equivalent values determined by gel permeation chromatography (GPC) (standard substance: polystyrene).

The flash point of the complex ester may be 150°C or higher, 180°C or higher, or 200°C or higher, and may be 350°C or lower, 300°C or lower, or 280°C or lower. The flash point herein is measured in accordance with the Cleveland Open Cup (COC) method described in JIS K2265-4:2007.

The kinematic viscosity of the complex ester at 40°C may be 20 mm²/s or more, 30 mm²/s or more, or 40 mm²/s or more, and may be 500 mm²/s or less, 300 mm²/s or less, or 200 mm²/s or less. The kinematic viscosity of the complex ester at 100°C may be 4 mm²/s or more, 8 mm²/s or more, or 10 mm²/s or more, and may be 50 mm²/s or less, 30 mm²/s or less, or 25 mm²/s or less. The viscosity index of the complex ester may be 120 or higher, 140 or higher, or 160 or higher, and may be 400 or lower, 300 or lower, or 200 or lower. The kinematic viscosity and viscosity index herein each refer to the values measured in accordance with JIS K2283:2000.

The acid value of the complex ester may be 1 mgKOH/g or less, 0.1 mgKOH/g or less, or 0.05 mgKOH/g or less. The acid value herein refers to the value measured in accordance with JIS K2501:2003.

The hydroxyl value of the complex ester may be 40 mgKOH/g or less, 15 mgKOH/g or less, 10 mgKOH/g or less, or 5 mgKOH/g or less. The hydroxyl value herein refers to the value measured in accordance with JIS K0070:1992.

The pour point of the complex ester may be -10°C or lower, - 20°C or lower, or -30°C or lower, and may be -60°C or higher. The pour point herein refers to the value measured in accordance with JIS K2269:1987.

The volume resistivity of the complex ester may be 0.0001 TΩ·m or higher, 0.001 TΩ·m or higher, or 0.01 TΩ·m or higher. The volume resistivity herein refers to the value at 25°C measured in accordance with JIS C2101:1999.

The complex ester described above is suitable for use in a refrigerating machine oil and is suitably used as a base oil for a refrigerating machine oil. In other words, another embodiment of the present invention may be a refrigerating machine oil comprising the above complex ester, or a base oil for refrigerating machine oil comprising the above complex ester.

In the refrigerating machine oil, the content of the above complex ester may be 10% by mass or more, 20% by mass or more, 30% by mass or more, 40% by mass or more, 50% by mass or more, 60% by mass or more, 70% by mass or more, 80% by mass or more, or 90% by mass or more, and may be 99% by mass or less, 90% by mass or less, 80% by mass or less, 70% by mass or less, 60% by mass or less, 50% by mass or less, 40% by mass or less, or 30% by mass or less, based the total amount of the refrigerating machine oil.

The refrigerating machine oil may further comprise an additional base oil in addition to the above complex ester. Examples of the additional base oil include known base oils such as oxygen-containing oils other than the above complex ester, and hydrocarbon oils. Examples of oxygen-containing oils include esters other than the above complex ester and ethers. Examples of the esters include polyol esters (excluding the above complex ester). Examples of the ethers include polyalkylene glycols and polyvinyl ethers. Examples of the hydrocarbon oils include mineral oil, alkylbenzene, alkylnaphthalene, poly-α-olefin, polybutene, and ethylene-α-olefin copolymers.

It is preferable that the refrigerating machine oil further comprises, in addition to the above complex ester, a polyol ester (excluding the above complex ester). Examples of such polyol esters include an ester of a polyhydric alcohol and a fatty acid.

The polyhydric alcohol may be a polyhydric alcohol having 2 to 6 hydroxyl groups. The number of carbon atoms in the polyhydric alcohol may be 4 or more, or 5 or more, and may be 12 or less, or 10 or less. Examples of the polyhydric alcohol include neopentyl glycol, trimethylolethane, trimethylolpropane, trimethylolbutane, di(trimethylolpropane), tri(trimethylolpropane), pentaerythritol, and dipentaerythritol. The polyhydric alcohol preferably comprises pentaerythritol, and may also comprise pentaerythritol and dipentaerythritol.

The fatty acid may be linear or branched, and may be saturated or unsaturated. The number of carbon atoms in the fatty acid may be 4 or more, or 5 or more, and may be 20 or less, 18 or less, or 9 or less. Examples of the fatty acid include 2-methylpropanoic acid, 2-methylbutanoic acid, 2-methylpentanoic acid, 2-methylhexanoic acid, 2-ethylpentanoic acid, 2-methylheptanoic acid, 2-ethylhexanoic acid, 3,5,5-trimethylhexanoic acid, oleic acid, stearic acid, and 2-ethylhexadecanoic acid. The fatty acid preferably comprises at least one selected from the group consisting of 2-methylpropanoic acid, 2-ethylhexanoic acid, and 3,5,5-trimethylhexanoic acid.

The content of the polyol ester in the refrigerating machine oil may be 10% by mass or more, 20% by mass or more, 30% by mass or more, 40% by mass or more, 50% by mass or more, 60% by mass or more, 70% by mass or more, 80% by mass or more, or 90% by mass or more, and may be 99% by mass or less, 90% by mass or less, 80% by mass or less, 70% by mass or less, 60% by mass or less, 50% by mass or less, 40% by mass or less, or 30% by mass or less, based on the total amount of the refrigerating machine oil.

The refrigerating machine oil may further comprise additives. Examples of additives include antiwear agents, antioxidants, acid scavengers, oxygen scavengers, oiliness agents, metal deactivators, viscosity index improvers, pour point depressants, and detergent dispersants. The total content of the additives may be, for example, 0.5% by mass or more, 1% by mass or more, or 2% by mass or more, and may be 5% by mass or more, 4% by mass or less, or 3% by mass or less, based on the total amount of the refrigerating machine oil.

In a refrigerating machine, the refrigerating machine oil may be present as a working fluid composition mixed with a refrigerant. That is, in one embodiment, the refrigerating machine oil is used together with a refrigerant. Another embodiment of the present invention is a working fluid composition comprising the above refrigerating machine oil and a refrigerant.

As the refrigerating machine oil is used together with a refrigerant, the kinematic viscosity can decrease substantially, making it more difficult to maintain an oil film on sliding parts. It is therefore required that the oil maintains a good balance between solubility with the refrigerant and properties for ensuring the oil film. In one embodiment, the refrigerating machine oil comprising the above-described complex ester can maintain a high balance between refrigerant solubility and refrigerant dissolution viscosity.

Particularly, the refrigerating machine oil comprising the above complex ester and a polyol ester can achieve both refrigerant solubility (especially solubility with R32) and refrigerant dissolution viscosity. By contrast, for example, the refrigerating machine oil comprising a polyol ester but not comprising the above complex ester tends to show a trade-off relationship between refrigerant solubility and refrigerant dissolution viscosity. That is, the refrigerant machine oil tends to show poor refrigerant dissolution viscosity even if its refrigerant solubility (especially solubility with R32) is good; conversely, the refrigerating machine oil tends to show poor refrigerant solubility (especially solubility with R32) even if the refrigerant dissolution viscosity is high. Also, for example, in the case of using a conventional complex ester comprising neopentyl glycol or a straight-chain divalent alcohol, the balance between refrigerant solubility (especially solubility with R32) and refrigerant dissolution viscosity has not necessarily been sufficient. By adopting the above complex ester as the base oil, it is possible to mitigate this trade-off relationship and obtain a fluid composition with a highly balanced relationship between refrigerant solubility and refrigerant dissolution viscosity.

For instance, in one embodiment, (for any refrigerant) the maximum value of the lower-temperature-side two layer separation temperature is preferably -2°C or lower, and the refrigerant dissolution viscosity at 80°C and 3.4 MPa is preferably 2.5 mm²/s or more. The maximum value of the lower-temperature-side two layer separation temperature is preferably -5°C or lower, more preferably -10°C or lower, and the refrigerant dissolution viscosity is preferably 2.6 mm²/s or more, 2.7 mm²/s or more, 2.8 mm²/s or more, or 2.9 mm²/s or more, and more preferably 3.0 mm²/s or more, 3.1 mm²/s or more, 3.2 mm²/s or more, or 3.3 mm²/s or more.

In one embodiment, with respect to R32, it is possible to achieve an excellent balance where the maximum value of the lower-temperature-side two layer separation temperature is -2°C or lower, preferably -5°C or lower, and the refrigerant dissolution viscosity at 80°C and 3.4 MPa is 2.5 mm²/s or more, 2.6 mm²/s or more, 2.7 mm²/s or more, or 2.9 mm²/s or more, preferably 3.0 mm²/s or more, 3.1 mm²/s or more, 3.2 mm²/s or more, or 3.3 mm²/s or more.

In one embodiment, when using a mixed refrigerant containing R32, it is possible to significantly lower the low-temperature-side two layer separation temperature without greatly reducing refrigerant dissolution viscosity, or to increase the refrigerant dissolution viscosity without significantly raising the low-temperature-side two layer separation temperature.

Specifically, in the case of a working fluid composition comprising R410A (R32/R125 = 50/50) and the refrigerating machine oil, it is possible to keep the refrigerant dissolution viscosity at 80°C and 3.4 MPa at, for example, 2.4 mm²/s or more or 2.5 mm²/s or more, preferably 2.6 mm²/s or more, 2.7 mm²/s or more, 2.8 mm²/s or more, 2.9 mm²/s or more, or 3.0 mm²/s or more, while keeping the maximum value of the lower-temperature-side two layer separation temperature - 5°C or lower, and preferably -20°C or lower, -30°C or lower, -40°C or lower, or -50°C or lower.

In the case of a working fluid composition comprising R454B (R32/HFO1234yf = 68.9/31.1) and the refrigerating machine oil, it is possible to keep the refrigerant dissolution viscosity at 80°C and 3.4 MPa at, for example, 2.4 mm²/s or more or 2.5 mm²/s or more, while keeping the maximum value of the lower-temperature-side two layer separation temperature, for example, -5°C or lower, preferably -20°C or lower, -30°C or lower, -40°C or lower, or -50°C or lower.

As described above, the refrigerating machine oil in the present embodiment is useful as a multipurpose oil, at least, for R32 alone, and for at least one R32-containing mixed refrigerants selected from R410A, R454B, and the like. In such an R32-containing mixed refrigerant, the R32 content is preferably 15% by mass or more, 40% by mass or more, or 60% by mass or more, and is preferably 90% by mass or less, 80% by mass or less, or 70% by mass or less, based on the total amount of the mixed refrigerant.

Examples of the refrigerant include saturated fluorocarbons (also called HFC), unsaturated fluorocarbons (also called HFO), hydrocarbons, fluorine-containing ethers, bis(trifluoromethyl) sulfide, trifluoroiodomethane, ammonia, and carbon dioxide (R744). In one embodiment, the refrigerant comprises a saturated fluorocarbon.

Examples of the saturated fluorocarbon include saturated fluorocarbons preferably having 1 to 3 carbon atoms, more preferably 1 to 2 carbon atoms. Specific examples of the saturated fluorocarbon include difluoromethane (R32), trifluoromethane (R23), pentafluoroethane (R125), 1,1,2,2-tetrafluoroethane (R134), 1,1,1,2-tetrafluoroethane (R134a), 1,1,1-trifluoroethane (R143a), 1,1-difluoroethane (R152a), fluoroethane (R161), 1,1,1,2,3,3,3-heptafluoropropane (R227ea), 1,1,1,2,3,3-hexafluoropropane (R236ea), 1,1,1,3,3,3-hexafluoropropane (R236fa), 1,1,1,3,3-pentafluoropropane (R245fa), and 1,1,1,3,3-pentafluorobutane (R365mfc). The saturated fluorocarbon preferably comprises difluoromethane (R32).

The preferable unsaturated fluorocarbons are fluorinated olefins having 2 to 4 carbon atoms selected from fluoroethylenes, fluoropropenes, and fluorobutenes. Examples include fluoropropenes having 1 to 5 fluorine atoms and 3 carbon atoms, such as 1,2,3,3,3-pentafluoropropene (HFO-1225ye), 1,3,3,3-tetrafluoropropene (HFO-1234ze), 2,3,3,3-tetrafluoropropene (HFO-1234yf), 1,2,3,3-tetrafluoropropene (HFO-1234ye), and 3,3,3-trifluoropropene (HFO-1243zf); fluoroethylenes having 1 to 3 fluorine atoms and 2 carbon atoms, such as monofluoroethylene (HFO-1141), 1,1-difluoroethylene (HFO-1132a), (E)-1,2-difluoroethylene (HFO-1132(E)), (Z)-1,2-difluoroethylene (HFO-1132(Z)), and 1,1,2-trifluoroethylene (R1123); fluorobutenes having 1 to 7 fluorine atoms and 4 carbon atoms, such as (E)-1,1,1,4,4,4-hexafluoro-2-butene (R1336mzz(E)) and (Z)-1,1,1,4,4,4-hexafluoro-2-butene (R1336mzz(Z)); and chlorine- and fluorine-containing unsaturated fluorocarbons, such as 1-chloro-2,2-difluoroethylene (HCFO-1122), (Z)-1-chloro-2,3,3,3-tetrafluoropropene (HCFO-1224yd(Z)), and (E)-1-chloro-2,3,3,3-tetrafluoropropene (HCFO-1224yd(E)). The unsaturated fluorocarbon is preferably at least one selected from HFO-1225ye, HFO-1234ze, and HFO-1234yf, and more preferably HFO-1234yf.

The hydrocarbon is preferably a hydrocarbon having 1 to 5 carbon atoms, more preferably 2 to 4 carbon atoms. Examples of the hydrocarbon include methane, ethylene, ethane, propylene, propane (R290), cyclopropane, normal butane, isobutane, cyclobutane, methylcyclopropane, 2-methylbutane, and normal pentane. The hydrocarbon is preferably at least one selected from the group consisting of propane, normal butane, isobutane, and 2-methylbutane.

In one embodiment, the refrigerant may comprise a saturated fluorocarbon (HFC). In one embodiment, the refrigerant may consist of one or more saturated fluorocarbons (HFC). Examples of such refrigerants include R32 alone; R23 alone; R134a alone; R125 alone; a mixture of R134a/R32 in 60-80% by mass / 40-20% by mass; a mixture of R32/R125 in 40-70% by mass / 60-30% by mass; a mixture of R125/R143a in 40-60% by mass / 60-40% by mass; a mixture of R134a/R32/R125 in 60% by mass / 30% by mass / 10% by mass; a mixture of R134a/R32/R125 in 40-70% by mass / 15-35% by mass / *5-*40% by mass; a mixture of R125/R134a/R143a in 35-55% by mass / 1-15% by mass / 40-60% by mass. More specific examples include a mixture of R134a/R32 = 70/30% by mass; a mixture of R32/R125 = 60/40% by mass; a mixture of R32/R125 = 50/50% by mass (R410A); a mixture of R32/R125 = 45/55% by mass (R410B); a mixture of R125/R143a = 50/50% by mass (R507C); a mixture of R32/R125/R134a = 30/10/60% by mass; a mixture of R32/R125/R134a = 23/25/52% by mass (R407C); a mixture of R32/R125/R134a = 25/15/60% by mass (R407E); and a mixture of R125/R134a/R143a = 44/4/52% by mass (R404A).

In one embodiment, the refrigerant may comprise both a saturated fluorocarbon (HFC) and an unsaturated fluorocarbon (HFO). Examples of such refrigerants include a mixture of R32/R134a/R125/HFO-1234yf/HFO-1234ze = 26/21/26/20/7% by mass (R448A); a mixture of R32/R134a/R125/HFO-1234yf = 24.3/25.7/24.7/25.3% by mass (R449A); a mixture of R32/R125/HFO-1234yf = 67/7/26% by mass (R452B); a mixture of R32/HFO-1234yf = 68.9/31.1% by mass (R454B); a mixture of R32/R125/HFO-1234ze = 68/3.5/28.5% by mass (R447A); a mixture of R32/R125/HFO-1234ze = 68/8/24% by mass (R447B); a mixture of R32/HFO-1234ze/R600a = 68/29/3% by mass (R446A); a mixture of R134a/HFO-1234yf= 44/56% by mass (R513A); a mixture of R32/HFO-1123 (for example, R32/HFO-1123 = 60-40% by mass/40-60% by mass); a mixture of R32/R152a/HFO-1234ze = 12/5/83% by mass (R444A); a mixture of R134a/HFO-1234ze/R744 = 9/85/6% by mass (R445A); a mixture of R134a/HFO-1234ze/R744 = 10.56/83.73/5.71% by mass (AC6A); a mixture of R32/HFO-1234yf/R744 = 21.5/75.5/3% by mass (R455A).

The refrigerant may also be a mixed refrigerant comprising other unsaturated fluorocarbon(s). Examples of such mixed refrigerants include R444B, R448B, R449B, R449C, R450A, R451A, R451B, R452A, R452C, R454A, R454C, R456A, R457A, R457B, R457C, R457D, R459A, R459B, R460A, R460B, R460C, R463A, R463A-J, R464A, R465A, R468A, R468B, R468C, R470A, R470B, R471A, R473A, R474A, R475A, R476A, R479A, R480A, R481A, R482A, R513B, R514A, R515A, R515B, and R516A.

The content of the refrigerating machine oil in the working fluid composition may be 1 part by mass or more or 2 parts by mass or more and may be 500 parts by mass or less or 400 parts by mass or less, relative to 100 parts by mass of the refrigerant.

The refrigerating machine oil and working fluid composition for a refrigerating machine oil can be suitably used for refrigerating machines such as air conditioners, refrigerators, open or closed car air conditioners, dehumidifiers, water heaters, freezers, refrigerated warehouses, vending machines, showcases, and chemical plants, which have reciprocating or rotary hermetic compressors, and refrigerating machines having centrifugal compressors.

### Examples

Hereinafter, the present invention will be described in more detail based on examples, but the present invention is not limited to the following examples.

### (Example 1-1)

An ester intermediate was obtained by the reaction of 2 moles of adipic acid with 1.24 moles of 1,2-propanediol. Subsequently, 1.52 moles of 2-ethylhexanol was further reacted with the ester intermediate. After that, in order to achieve a low acid value and a low hydroxyl value, the unreacted materials and trace impurities were removed by distillation and through the use of an adsorbent, thus Complex Ester 1 was obtained.

### (Example 1-2)

Complex Ester 2 was obtained in the same manner as in Example 1-1 except that the ratio of the raw materials was changed to 2 moles of adipic acid, 1.51 moles of 1,2-propanediol, and 0.93 moles of 2-ethylhexanol.

### (Example 1-3)

Complex Ester 3 was obtained in the same manner as in Example 1-1 except that the ratio of the raw materials was changed to 2 moles of adipic acid, 1.64 moles of 1,2-propanediol, and 0.8 moles of 2-ethylhexanol.

### (Example 1-4)

Complex Ester 4 was obtained in the same manner as in Example 1-1 except that the raw materials were changed to 2 moles of adipic acid, 1.33 moles of 1,2-propanediol, and 1.38 moles of 1-butanol.

### (Example 1-5)

Complex Ester 5 was obtained in the same manner as in Example 1-1 except that the raw materials were changed to 2 moles of adipic acid, 1.14 moles of 3-methyl-1,5-pentanediol, and 1.62 moles of 1-butanol.

### (Example 1-6)

Complex Ester 6 was obtained in the same manner as in Example 1-1 except that the raw materials were changed to 2 moles of adipic acid, 1.33 moles of 2-methyl-1,3-propanediol, and 1.38 moles of 1-butanol.

These Complex Esters 1 to 6 contained compounds represented by the above formula (2) and having m = 1 to 5, as main components.

### (Comparative Example 1-1)

Complex Ester 7 was obtained by reacting 2 moles of adipic acid, 0.51 moles of neopentyl glycol, 0.56 moles of 1,4-butanediol, and 1.58 moles of 3,5,5-trimethylhexanol, and by removing the unreacted materials and trace impurities by distillation and through the use of an adsorbent in order to achieve a low acid value.

### (Comparative Example 1-2)

Complex Ester 8 was obtained in the same manner as in Comparative Example 1-1 except that the raw materials were changed to 2 moles of adipic acid, 1.3 moles of neopentyl glycol, 0.19 moles of 1,4-butanediol, and 1.16 moles of 3,5,5-trimethylhexanol.

### (Comparative Example 1-3)

Complex Ester 9 was obtained in the same manner as in Comparative Example 1-1 except that 1,2-propanediol was changed to 1,2-ethanediol.

Table 1 shows the physical properties of each of the obtained complex esters and the results of evaluation of low-temperature stability described below. However, with regard to Complex Ester 9 of Comparative Example 1-3 (using 1,2-ethanediol as divalent alcohol), the physical properties and evaluation results for low-temperature stability are not shown, since the complex ester solidified at room temperature.

### (Evaluation of Low-Temperature Stability)

Each complex ester of 10 g was placed in a test tube and stored for two weeks at -40°C. The appearance of any precipitate in the complex ester was then observed, and the low-temperature stability was evaluated according to the following criteria:
A: No precipitation was observed.
B: A small amount of precipitation was observed, but most of the sample was transparent.
C: Precipitation was clearly observed, and the entire sample was cloudy.

**[Table 1]**

| | | Ex.1-1 | Ex.1-2 | Ex.1-3 | Ex.1-4 | Ex.1.5 | Ex.1-6 | Comp. Ex. 1-1 | Comp. Ex. 1-2 |
|---|---|---|---|---|---|---|---|---|---|
| Type of divalent alcohol | | 1,2-propanediol | 1,2-propanediol | 1,2-propanediol | 1,2-propanediol | 3-methyl-1,5-pentanediol | 2-methyl-1,3-propanediol | Neopentyl glycol / 1,4-butanediol | Neopentyl glycol / 1,4-butanediol |
| Physical properties | Density | 1.024 | 1.061 | 1.073 | 1.076 | 1.040 | 1.058 | 1.00 | 1.017 |
| | Number average molecular weight Mn | 586 | 903 | 1090 | 681 | 671 | 640 | - | 714 |
| | Weight average molecular weight Mw | 1060 | 1740 | 2140 | 1280 | 1380 | 1270 | - | 1330 |
| | Dispersity Mw/Mn | 1.81 | 1.93 | 1.96 | 1.88 | 2.06 | 1.98 | - | 1.86 |
| | Flash point (°C) | 258 | 272 | 252 | 196 | 196 | 216 | 242 | 240 |
| | Kinematic viscosity at 40° C (mm²/s) | 53.4 | 155.3 | 221.0 | 54.7 | 45.9 | 49.4 | 68.26 | 142 |
| | Kinematic viscosity at 100° C(mm²/s) | 9.1 | 19.8 | 25.6 | 9.68 | 9.72 | 9.48 | 11.12 | 18.16 |
| | Viscosity Index | 152 | 147 | 147 | 163 | 204 | 180 | 155 | 143 |
| | Acid value (mgKOH/g) | <0.01 | 0.01 | 0.02 | 0.01 | <0.01 | <0.01 | <0.01 | 0.02 |
| | Hydroxyl value (mgKOH/g) | 1.9 | 2.6 | 3.9 | 4.1 | 3.0 | 3.3 | 30 | 25 |
| | Pour point (°C) | -37.5 | -37.5 | -35 | -35.0 | -45.0 | -42.5 | <-45 | -42.5 |
| | Volume resistivity (TΩ·m) | 0.025 | 0.05 | 0.03 | 0.004 | 0.01 | 0.002 | 0.01 | 0.069 |
| Low-temperature stability | | A | A | A | A | A | A | C | A |

### (Examples 2-1 to 2-2 and Comparative Examples 2-1 to 2-2)

Refrigerating machine oils having the compositions (mass%, based on the total amount of the refrigerating machine oil) shown in Table 2 were prepared by using Complex Esters 2, 3, and 8 described above, and the polyol ester described below.
Polyol ester: A polyol ester of pentaerythritol and a mixture of 2-methylpropanoic acid/3,5,5-trimethylhexanoic acid (molar ratio: 37/63) (kinematic viscosity of 68 mm²/s at 40°C).

For each of the obtained refrigerating machine oils, measurements of two layer separation temperature and refrigerant dissolution viscosity were carried out by the procedure below. The results are shown in Table 2.

### (Measurement of Two Layer Separation Temperature)

At oil rates (OR = [mass of refrigerating machine oil (g) / total mass of the refrigerating machine oil and refrigerant (R32) (g)] × 100) of 10%, 15%, 20%, and 30%, respectively, the low-temperature-side two layer separation temperature was measured.

### (Measurement of Refrigerant Dissolution Viscosity)

First, a pressure-resistant container (volume: 400 cm³) equipped with a vibrational viscometer, a thermometer, and a pressure gauge was filled with 100 g of refrigerating machine oil. After vacuum degassing the inside of the pressure-resistant container, the refrigerant (R32) was filled, and the temperature and pressure inside the pressure-resistant container were adjusted to 80°C and an absolute pressure of 3.4 MPa, respectively, to prepare a working fluid composition consisting of the refrigerant and the refrigerating machine oil. The absolute viscosity and volume of the working fluid composition at that time were measured. The refrigerant vapor density, the amounts of the charged oil and refrigerant, and the mass of the working fluid were used to determine the density of the working fluid, from which the refrigerant dissolution viscosity (mm²/s) was calculated.

**[Table 2]**

| | | | Ex. 2-1 | Ex. 2-2 | Ex. 2-3 | Comp. Ex. 2-1 | Comp. Ex. 2-2 |
|---|---|---|---|---|---|---|---|
| Composition | Complex Ester | 2 | 20 | 10 | - | - | - |
| | | 3 | - | - | 15 | - | - |
| | | 8 | - | - | - | 20 | - |
| | Polyol Ester | | 80 | 90 | 85 | 80 | 100 |
| Measurement results (R32) | Two layer separation temperature (°C) | OR=10% | -11 | -19 | -12 | -7 | -21 |
| | | OR=15% | -7 (max.) | -13 | -9 | -1 (max.) | -16 |
| | | OR=20% | -7 | -12 (max.) | -8 (max.) | -2 | -15 (max.) |
| | | OR=30% | -8 | -13 | -12 | -6 | -17 |
| | Refrigerant dissolution viscosity (mm²/s) | | 3.4 | 2.7 | 2.9 | 3.1 | 2.4 |

From the results in Table 2, it can be seen that, in the case where a complex ester in which the divalent alcohol consists of a branched divalent alcohol having no quaternary carbon was use (for example, Example 2-1), the balance between refrigerant compatibility and refrigerant dissolution viscosity was improved, compared with the case where a conventional complex ester was used (Comparative Example 2-1).

In Example 2-1 and Comparative Example 2-2, the maximum two layer separation temperature and the refrigerant dissolution viscosity were similarly measured when the refrigerant was changed from R32 to R410A or R454B. As shown by the results below, even when R32-containing mixed refrigerants were used instead of R32 alone, employing a complex ester in which the divalent alcohol consists of a branched divalent alcohol having no quaternary carbon led to higher refrigerant dissolution viscosity and an improved balance with refrigerant compatibility and refrigerant dissolution viscosity. (When R410A was used)

### • Example 2-1

Two layer separation temperature (maximum): -55°C;
Refrigerant dissolution viscosity: 3.0 mm²/s

### • Comparative Example 2-2

Two layer separation temperature (maximum): -51°C;
Refrigerant dissolution viscosity: 2.5 mm²/s
(When R454B was used)

### • Example 2-1

Two layer separation temperature (maximum): -50°C;
Refrigerant dissolution viscosity: 2.5 mm²/s

### • Comparative Example 2-2

Two layer separation temperature (maximum): -53°C;
Refrigerant dissolution viscosity: 2.2 mm²/s.

## Claims

1. A complex ester of a divalent carboxylic acid, a divalent alcohol, and at least one selected from a monohydric alcohol and a monocarboxylic acid,
wherein the divalent alcohol consists of a branched divalent alcohol having no quaternary carbon.

2. A refrigerating machine oil comprising:
a complex ester of a divalent carboxylic acid, a divalent alcohol, and at least one selected from a monohydric alcohol and a monocarboxylic acid,
wherein the divalent alcohol consists of a branched divalent alcohol having no quaternary carbon.

3. The refrigerating machine oil according to claim 2, further comprising a polyol ester of a polyhydric alcohol and a fatty acid.

4. A working fluid composition comprising:
the refrigerating machine oil according to claim 2 or 3, and
a refrigerant.

5. The working fluid composition according to claim 4, wherein the refrigerant comprises difluoromethane.
